# EUROPÄISCHE PATENTANMELDUNG

(11) **EP 2 354 122 A1**
(43) Veröffentlichungstag der Anmeldung: **10.08.2011**
(21) Anmeldenummer: 10000672.5
(22) Anmeldetag: 23.01.2010
(51) Int. Cl.: C07C 309/17, C11D 1/12

(54) **Sulfosuccinate**

(71) Anmelder: Cognis IP Management GmbH, 40589 Düsseldorf (DE)
(72) Erfinder: Busch, Stefan, DE-40597 Düsseldorf (DE); Birnbrich, Paul, DE-42719 Solingen (DE); Mahnke, Eike Ulf, DE-42553 Velbert (DE); Wick, Anja, DE-40723 Hilden (DE)

(57) **Zusammenfassung**

Sulfosuccinate der allgemeinen Formel (I), 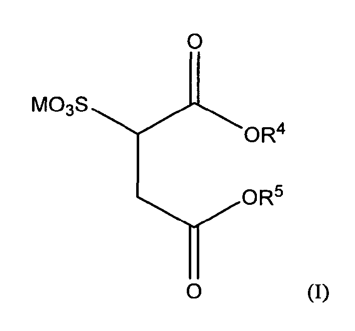 worin M Wasserstoff oder ein Kation und die Reste R⁴ und R⁵- unabhängig voneinander - Wasserstoff oder ein Kation oder eine Alkylgruppe bedeuten, wobei höchstens einer der Reste R⁴ oder R⁵ Wasserstoff oder ein Kation ist, wobei diese Sulfosuccinate mindestens einen Alkohol-Baustein enthalten, der den Monoalkoholen mit insgesamt 8 bis 36 C-Atomen zuzurechnen ist, mit der Maßgabe, dass es sich bei diesen Monoalkoholen um Guerbet-Alkohole (GA) handelt, die mindestens zwei Verzweigungen pro Molekül enthalten, eignen sich als Tenside mit verbesserter dynamischer Oberflächenspannung.

## Beschreibung

### Gebiet der Erfindung

Die Erfindung betrifft Sulfosuccinate spezieller Stuktur und Zusammensetzungen enthaltend diese Sulfosuccinate und Wasser.

### Stand der Technik

Guerbet-Alkohole sind spezielle verzweigte Alkohole. Es handelt sich um primäre Alkohole, die in 2-Position verzweigt sind. Guerbet-Alkohole sind dem Fachmann bekannt, manche sind seit langem kommerziell erhältlich. Sie werden durch die so genannte Guerbet-Reaktion gewonnen, eine seit etwa 100 Jahren bekannte Dimerisierungs-Reaktion, die durch folgendes Formelschema (R* bedeutet darin eine aliphatische Gruppe) umschrieben werden kann:

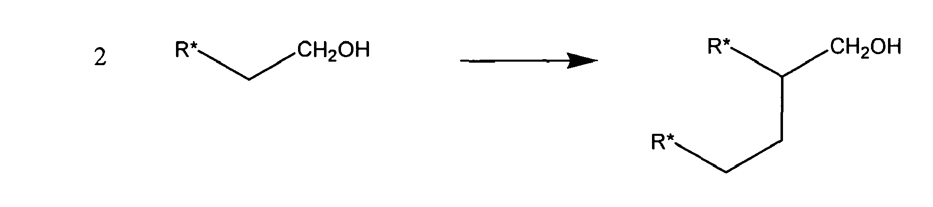

Im Rahmen der klassischen Guerbet-Reaktion wird ein primärer oder sekundärer Alkohol in einen Alkohol etwa doppelten Molekulargewichts überführt, der in 2-Position alkyliert ist. So wird etwa n-Butanol in 2-Ethyl-hexan-1-ol überführt, Hexan-1-ol in 2-Butyloctan-1-ol und Octan-1-ol in 2-Hexyl-decan-1-ol.

Die für die Guerbet-Reaktion eingesetzten primären oder sekundären Alkohole tragen an dem C-Atom, das dem C-Atom mit der OH-Gruppe unmittelbar benachbart ist, mindestens ein Wasserstoffatom - in vielen Fällen tragen sie zwei Wasserstoffatome, d.h. dem C-Atom mit der OH-Gruppe ist dann eine Methylengruppe unmittelbar benachbart.

Die Guerbet-Reaktion verläuft typischerweise in Gegenwart einer Base bei erhöhter Temperatur unter Wasserabspaltung und stellt eine einfache Möglichkeit dar, lineare Alkohole in verzweigte Alkohole zu überführen. Typischerweise setzt man nur einen einzigen Alkohol bei der Guerbet-Reaktion ein. Es ist jedoch auch möglich, zwei unterschiedliche Alkohole einzusetzen; in diesem Falle spricht man von einer gemischten Guerbet-Reaktion.

Im obigen Formelschema bedeuten die Reste R* jeweils eine aliphatische Gruppe. Typischerweise handelt es sich um eine lineare und gesättigte aliphatische Gruppe, d.h. man setzt etwa Fettalkohole wie Octanol oder Decanol als Ausgangs-Alkohole für die Guerbet-Reaktion ein. Bisweilen handelt es sich bei R* um eine gesättigte cycloaliphatische Gruppe, d.h. man setzt etwa Alkohole wie Cyclopentanol oder Cyclohexanol als Ausgangs-Alkohole für die Guerbet-Reaktion ein.

Sulfosuccinate sind eine seit langem bekannte Tensidklasse.

Bezüglich der sogenannten statischen Oberflächenspannung werden die Eigenschaften der Sulfosuccinate in hohem Maße durch deren Alkoholbausteine bestimmt. Mit größerwerdenden Alkylresten dieser Alkoholbausteine sinkt das Verhältnis von hydrophilen zu lipophilen Molekülteilen (vergleichbar mit dem für nichtionische Tenside von Griffin vorgeschlagenen HLB-Wert (= hydrophile-lipophile-balance)). Bei der Variation der Größe der Alkylreste der Alkoholbausteine von Sulfosuccinaten wird ein Optimum für die tensidische Aktivität durchlaufen. So läßt sich die maximal erreichbare Verringerung der Oberflächenspannung einer wäßrigen Lösung von Didecylsulfosuccinaten schon bei deutlich geringeren Konzentrationen erreichen als mit Dioctylsulfosuccinaten, während Ditridecylsulfosuccinate wieder eine deutlich schwächere Oberflächenaktivität aufweisen.

Eine weitere wichtige Größe zur Bewertung von Tensiden stellt die dynamische Oberflächenspannung dar. Diese gibt an, wie schnell ein Tensid eine neu entstehende Oberfläche belegen kann. Dies ist besonders bei schnellaufenden Prozessen wie dem Lackauftrag bei Druckerzeugnissen wichtig. Generell wirkt sich eine Vergrößerung des Alkylrests und damit die zunehmende Größe des Tensids ungünstig auf die dynamische Oberflächenspannung aus. Am Beispiel der Sulfosuccinate, die Isodecanol bzw. 2-Propylheptanol als Alkoholbausteine enthalten, läßt sich erkennen, dass bei Alkoholbausteinen mit gleicher Gesamtzahl an C-Atomen, die exakte Struktur der Alkoholbausteine einen Einfluß auf die dynamische Oberflächenspannung hat. Dabei gilt allerdings, dass eine verläßliche Berechnung bzw. Vorhersage der dynamischen Oberflächenspannung eines bestimmten Tensids derzeit nicht möglich ist, da verschiedene Prozesse wie Diffusion, Adsorption und Ausrichtung des Tensids oder bestimmter Molekülteile an der Oberfläche sehr genau berücksichtigt werden müssen.

Sulfosuccinate können lineare oder verzweigte Alkoholbausteine enthalten. Zu den kommerziell eingesetzten verzweigten Alkoholbausteinen gehören das methylverzweigte Isodecanol und Isotridecanol sowie das 2-Ethylhexanol. Dabei stellt 2-Ethylhexanol, obwohl es großtechnisch durch Aldolkondensation hergestellt wird, formal einen Guerbet-Alkohol dar, weil er durch Verknüpfung zweier Butanol-Moleküle durch Guerbet-Reaktion zugänglich ist. Auch 2-Propylheptanol ist ein Guerbet-Alkohol, weil er durch Verknüpfung zweier Pentanol-Moleküle durch Guerbet-Reaktion zugänglich ist. Das Sulfosuccinat von 2-Propylheptanol ist jedoch bislang nicht kommerziell verfügbar.

### Beschreibung der Erfindung

Aufgabe der Erfindung war es, Sulfosuccinate bereitzustellen, die gegenüber den aus dem Stand der Technik bekannten Sulfosuccinaten eine verbesserte dynamische Oberflächenspannung der entsprechenden wässrigen Lösungen aufweisen.

Der Begriff "Sulfosuccinate" ist dem Fachmann bekannt. Es handelt sich bei Sulfosuccinaten um Salze folgender allgemeiner Formel (I)

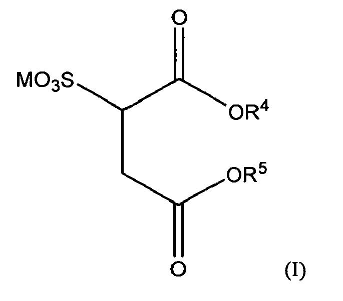

In der Formel (I) ist M Wasserstoff oder ein Kation; die Reste R⁴ und R⁵ bedeuten - unabhängig voneinander - Wasserstoff oder ein Kation oder eine Alkylgruppe, wobei höchstens einer der Reste R⁴ oder R⁵ Wasserstoff oder ein Kation bedeuten kann. Wie dem Fachmann bekannt spricht man von Monoalkylsulfosuccinaten, wenn nur einer der Reste R⁴ oder R⁵ eine Alkylgruppe bedeutet und von Dialkylsulfosuccinaten, wenn beide Reste R⁴ und R⁵ eine Alkylgruppe bedeuten.

Im Rahmen der vorliegenden Erfindung wird der Begriff "Sulfosuccinate" in dem vorstehend genannten Sinne verwendet, d.h. er umfasst sowohl Monoalkylsulfosuccinate als auch Dialkylsulfosuccinate. Da die Sulfosuccinate (I) Ester darstellen, kann man logischerweise für den Fall, dass die Reste R⁴ bzw. R⁵ Alkylgruppen sind, sagen, dass die Monoalkohole R⁴OH bzw. R⁵OH die Alkoholbausteine der Sulfosuccinate sind. Von dieser Vereinfachung der sprachlichen Ausdrucksweise wird im folgenden Gebrauch gemacht.

Gegenstand der vorliegenden Erfindung sind zunächst Sulfosuccinate der allgemeinen Formel (I), 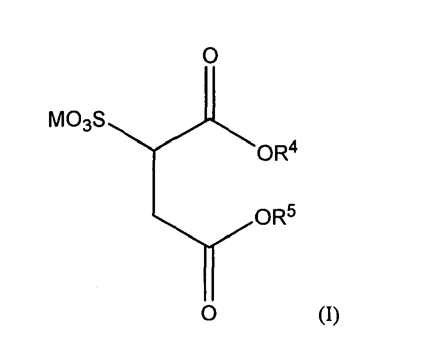 worin M Wasserstoff oder ein Kation und die Reste R⁴ und R⁵- unabhängig voneinander - Wasserstoff oder ein Kation oder eine Alkylgruppe bedeuten, wobei höchstens einer der Reste R⁴ oder R⁵ Wasserstoff oder ein Kation bedeuten kann, wobei diese Sulfosuccinate mindestens einen Alkohol-Baustein enthalten, der den Monoalkoholen (i) mit insgesamt 8 bis 36 C-Atomen zuzurechnen ist, mit der Maßgabe, dass es sich bei diesen Monoalkoholen um Guerbet-Alkohole (GA) handelt, die mindestens zwei Verzweigungen pro Molekül enthalten.

In einer Ausführungsform handelt es sich bei den Monoalkoholen um solche Guerbet-Alkohole (GA), die mindestens drei Verzweigungen pro Molekül enthalten.

Vorzugsweise handelt es sich bei den Monoalkoholen um solche Guerbet-Alkohole (GA), die drei Verzweigungen pro Molekül enthalten und die erhältlich sind, indem man als Ausgangs-Alkohole für die Guerbet-Reaktion zur Herstellung dieser Guerbet-Alkohole (GA) ausschließlich solche Monoalkohole mit 4 bis 18 C-Atomen einsetzt, die ausgewählt sind aus der Gruppe der primären und/oder sekundären Monoalkohole der Formel (MA), 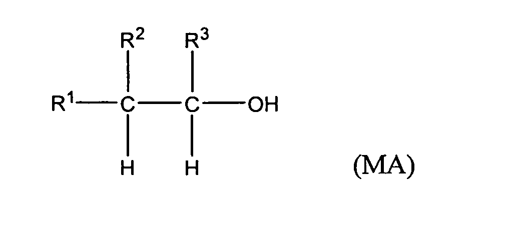 wobei gilt: (a) die Gesamtzahl der C-Atome der Verbindungen (MA) liegt im Bereich von 4 bis 18; (b) die Reste R¹, R² und R³ bedeuten Wasserstoff oder Alkylgruppen, die - unabhängig voneinander - linear oder verzweigt oder alicyclisch, gesättigt oder ungesättigt sein können; (c) die Reste R¹ und R² und/oder R¹ und R³ und/oder R² und R³ können miteinander verknüpft sein, d.h. Teil einer alicyclischen Substruktur sein; (d) die Verbindungen (MA) weisen mindestens eine Verzweigung auf.

### Zu den Monoalkoholen (i)

Wie oben ausgeführt handelt es sich bei den Alkoholbausteinen der Sulfosuccinate um Monoalkohole (i) mit insgesamt 8 bis 36 C-Atomen, mit der Maßgabe, dass es sich bei diesen Monoalkoholen um Guerbet-Alkohole (GA) handelt, die mindestens zwei Verzweigungen pro Molekül enthalten.

Der Begriff der Verzweigung ist hiebei wie folgt zu verstehen:
- C-Atome, die direkt mit 3 anderen C-Atomen verknüpft sind (tertiäre C-Atome), stellen eine Verzweigung dar.
- C-Atome, die direkt mit 4 anderen C-Atomen verknüpft sind (quartäre C-Atome), stellen zwei Verzweigungen dar.

Wenn die genannte Bedingung lautet, dass die Guerbet-Alkohole (GA) mindestens zwei Verzweigungen pro Molekül enthalten müssen, dann ist damit die Gesamtzahl der Verzweigungen im Molekül gemeint.

Was Guerbet-Alohole und die zu ihrer Herstellung angewandte Guerbet-Reaktion sind, ist dem Fachmann bekannt und wichtige Punkte hierzu wurden im Kapitel zum Stand der Technik bereits ausgeführt (siehe oben). Wie aus dem in diesem Zusammenhangdargestellten Formelschema (siehe oben) hervorgeht, besteht die Guerbet-Reaktion im Endeffekt darin, zwei Moleküle Ausgangsalkohol in einer Kondensationsreaktion miteinander zu verknüpfen, wobei das resultierende Molekül, also der Guerbet-Alkohol an derjenigen Stelle, an der die beiden ursprünglichen Ausgangs-Alkohol-Bausteine miteinander verknüpft sind, eine Verzweigung im Molekül enthält. Sofern R* im obigen Formelschema eine lineare aliphatische Gruppe ist, enthält der resultierende Guerbet-Alkohol eine Verzweigung im Molekül. Sofern R* im obigen Formelschema eine cycloaliphatische Gruppe ist, enthält der resultierende Guerbet-Alkohol zwei Verzweigungen im Molekül.

In einer bevorzugten Ausführungsform setzt man im Rahmen der vorliegenden Erfindung solche Guerbet-Alkohole (GA) ein, die mindestens drei Verzweigungen pro Molekül enthalten.

In einer besonders bevorzugten Ausführungsform setzt man im Rahmen der vorliegenden Erfindung solche Guerbet-Alkohole (GA) ein, die drei Verzweigungen pro Molekül enthalten und die erhältlich sind, indem man als Ausgangsalkohole für die Guerbet-Reaktion zur Herstellung dieser Guerbet-Alkohole (GA) ausschließlich solche Monoalkohole mit 4 bis 18 C-Atomen einsetzt, die ausgewählt sind aus der Gruppe der primären und/oder sekundären Monoalkohole der Formel (MA), 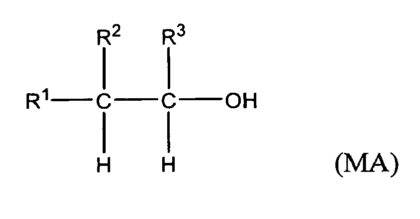 wobei gilt: (a) die Gesamtzahl der C-Atome der Verbindungen (MA) liegt im Bereich von 4 bis 18; (b) die Reste R¹, R² und R³ bedeuten Wasserstoff oder Alkylgruppen, die - unabhängig voneinander - linear oder verzweigt oder alicyclisch, gesättigt oder ungesättigt sein können; (c) die Reste R¹ und R² und/oder R¹ und R³ und/oder R² und R³ können miteinander verknüpft sein, d.h. Teil einer alicyclischen Substruktur sein; (d) die Verbindungen (MA) weisen mindestens eine Verzweigung auf.

Vorzugsweise sind dabei die Alkylgruppen der Verbindungen (MA) ausschließlich gesättigt.

Bei den Verbindungen (MA), deren Alkylreste ausschließlich gesättigt sind, gilt in einer bevorzugten Ausführungsform, dass die Gesamtzahl ihrer C-Atome im Bereich von 5 bis 10 liegt. Dabei sind Alkohole mit 5 C-Atomen und insbesondere Isomerengemische von Alkoholen mit 5 C-Atomen besonders bevorzugt.

3-Methyl-butan-1-ol ist als Monoalkohol (MA) ganz besonders bevorzugt. Er kann in reiner Form oder in Form technischer Mischungen und auch in Abmischung mit anderen Alkoholen der Formel (MA) als Ausgangs-Alkohol eingesetzt werden. Durch Guerbetreaktion entsteht beim Einsatz von 3-Methyl-butan-1-ol als Monoalkohol (MA) der folgende Guerbetalkohol (GA*) 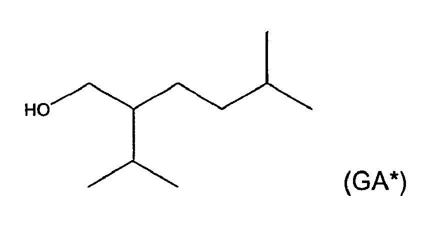 der als 2-Isopropyl-5-Methyl-hexan-1-ol bezeichnet werden kann.

Die Verbindungen (MA) können in reiner Form oder in Form technischer Produkte eingesetzt werden. Es ist auch möglich, Mischungen für die erfindungsgemäß durchzuführende Guerbetreaktion einzusetzen, die im wesentlichen ein oder mehrere Verbindungen (MA) neben weiteren Substanzen - wobei unverzweigte aliphatische Alkohole als weitere Substanzen ausgeschlossen sind - enthalten.

### Weitere Ausführungsformen

Wie oben ausgeführt hat M in der Formel (I) der Sulfosuccinate die Bedeutung Wasserstoff oder ein Kation. Dabei gilt selbstverständlich die Nebenbedingung, dass die Sulfosuccinate insgesamt elektrisch neutral sind.

In einer bevorzugten Ausführungsform wird M ausgewählt aus der Gruppe 1-wertiger Kationen, insbesondere aus der Gruppe der Alkalimetall-Kationen. Natrium, Kalium und Ammonium sind als Kationen M ganz besonders bevorzugt.

Wie oben ausgeführt kann es sich bei den Sulfosuccinaten (I) und Monoalkyl-und/oder Dialkylsulfosuccinate handeln. Dialkylsulfosuccinate sind dabei bevorzugt.

Dialkylsulfosuccinate, bei denen die Monoalkohole (MA) gesättigte, mindestens einfach verzweigte Monoalkohole mit 5 bis 10 C-Atomen sind, insbesondere 3-Methylbutan-1-ol, sind ganz besonders bevorzugt.

### Zusammensetzungen

Ein weiterer Erfindungsgegenstand sind Zusammensetzungen, enthaltend ein oder mehrere der oben näher erläuterten Sulfosuccinate (I) und Wasser.

### Verwendung

Desweiteren betrifft die Erfindung die Verwendung der Sulfosuccinate (I) als Tenside. Dabei haben die Untersuchungen der Anmelderin gezeigt, dass die erfindungsgemäßen Sulfosuccinate (I) sich gegenüber den aus dem Stand der Technik bekannten Sulfosuccinaten durch eine verbesserte dynamische Oberflächenspannung auszeichnen: Wie anhand der unten aufgeführten Beispiele ersichtlich, lassen sich mit den erfindungsgemäßen Sulfosuccinaten höhere Oberflächenbildungsraten ohne signifikanten Anstieg der Oberflächenspannung erreichen.

In weiteren Ausführungsformen betrifft die Erfindung die Verwendung der Sulfosuccinate (I) als Netzmittel, insbesondere in Lacken, sowie als Emulgator, insbesondere in der Emulsionspolymerisation.

### Beispiele

| | **Eingesetzte Substanzen** |
|---|---|
| Natriumdisulfit | von Fa. BASF |
| Maleinsäureanhydrid | von Fa. Sasol-Huntsman |
| Hydropalat 875 | anionisches Netzmittel (von Fa. Cognis) |
| C10-V1-OH | 2-Propylheptanol (Fa. BASF; 1-fach verzweigter Alkohol mit 10 C-Atomen) |
| C10-V3-OH | 3-fach-verzweigter Alkohol mit 10 C-Atomen, der wie folgt durch Guerbet-Reaktion aus 3-Methyl-1-butanol in Gegenwart von katalytischen Mengen Aldehyd, Alkali und Palladium auf Kohle hergestellt wurde: Ein Reaktionsgemisch aus 2500g 3-Methyl-1-Butanol (ein Isoamylalkohol), 75g 3-Methyl-1-Butanal, 4 g Pd/C (5% Pd/C 3610 von der Firma Johnson Matthey in 50% Wasser) wurde auf 180°C aufgeheizt, 320g KOH (50%ig) portionsweise zudosiert und mit einer Druck-rampe von 4,6 auf 1,4 bar 18 Stunden unter Rühren gefahren. Der erhaltene 3-fach verzweigte Alkohol wird unten auch als "Isoamylguerbetalkohol" bezeichnet. |

### Beispiele und Vergleichsbeispiele

### Beispiel 1 (zum Vergleich) = B1:

### Herstellung von Di-(2-propyrlheptyl)-maleinat

In einem 21-Vierhalskolben mit mechanischem Rührer, Heizung, Destillationsapparatur und Stickstoff/Vakuumanschluß wurden unter Stickstoffatmosphäre 392 g
- (4,0 mol) Maleinsäureanhydrid,
- 1330 g (8,4 mol) C10-V1-OH (2-Propylheptanol) und
- 8,6 g (0,05 mol) p-Toluolsulfonsäure-Monohydrat
   eingewogen und langsam auf 140 °C erhitzt. Das entstehende Reaktionswasser wurde destillativ abgetrennt. Sobald nur noch wenig Destillat überging (etwa 3,5 Stunden nach Reaktionsbeginn) wurde der Druck langsam auf bis zu 10 mbar gesenkt und 3 Stunden gehalten. Das erhaltene Produkt wies eine Restsäurezahl von 1,66 mg KOH/g auf.

### Beispiel 2 (zum Vergleich) = B2:

### Herstellung von Natrium-di-(2-propylheptyl)-sulfosuccinat

### (Lösung in Wasser/Ethanol)

Bei der erstmaligen Durchführung der Synthese gemäß Beispiel 2 wurde wie folgt verfahren:
In einen 11-Vierhalskolben mit mechanischem Rührer, Heizung, Rückflußkühler und Stickstoffeinleitung wurden
   - 574 g (1,45 mol) Di-(2-propylheptyl)-maleinat (hergestellt gemäß Beispiel 1),
   - 40 g Hydropalat 875
   - 142 g (0,75 mol) Natriumdisulfit und
   - 194 g vollentsalztes Wasser
      vorgelegt und der Ansatz bei 104 °C unter Rückfluß und leichtem Stickstoffstrom so lange gerührt, bis eine klare Lösung entstanden war (ca. 3 Stunden). Es wurde noch 10 Minuten nachgerührt. Im Produkt war kein Sulfit mehr nachweisbar. Beim Abkühlen vergelte/verfestigte sich das Produkt, was durch Zugabe eines Co-Solvens vermieden werden konnte: Hierzu wurden bei ca. 80 °C 50 g Ethanol zugesetzt.

Bei der zweiten Durchführung der Synthese gemäß Beispiel 2 wurde wie oben beschrieben verfahren, jedoch mit dem Unterschiede, dass anstelle von 40 g Hydropalat 875 40 g einer Lösung aus (a) 70% Di-(2-propylheptyl)-sulfosuccinat, (b) 20% Wasser und (c) 10% Ethanol eingesetzt wurden (Komponente (a) dieser Lösung stand bei der oben beschriebenen erstmaligen Durchführung der Synthese noch nicht zur Verfügung).

Das so erhaltene Produkt wies folgende Kennzahlen auf: Säurezahl: 0,18 mg KOH/g; Epton: 14,01 %; Trockenrückstand: 71,66 Gew.-%; Gehalt an Natriumsulfat: Gew.-0,32 %.

### Beispiel 3 (erfindungsgemäß) = B3:

### Herstellung von Di-(isoamylguerbet)-maleinat

In einem 0,51-Vierhalskolben mit mechanischem Rührer, Heizung, Destillationsapparatur mit Wasserabscheider und Stickstoff/Vakuumanschluß wurden unter Stickstoffatmosphäre
- 49,03 g (0,5 mol) Maleinsäureanhydrid,
- 162,5 g (1,0 mol) C10-V3-OH (Isoamylguerbetalkohol) und
- 1,04 g (0,01 mol) p-Toluolsulfonsäure-Monohydrat
   eingewogen und langsam auf 140 °C erhitzt. Das entstehende Reaktionswasser wurde destillativ abgetrennt. Sobald nur noch wenig Destillat überging (etwa 3,5 Stunden nach Reaktionsbeginn) wurde der Druck langsam auf bis zu 10 mbar gesenkt und 3 Stunden gehalten. Das erhaltene Produkt wies eine Restsäurezahl von 4,26 mg KOH/g auf.

### Beispiel 4 (erfindungsgemäß) = B4:

### Herstellung von Natrium-di-(isoamylguerbet)-sulfosuccinat (wäßrige Lösung)

In einen 0,5 1-Vierhalskolben mit mechanischem Rührer, Heizung, Rückflußkühler und Stickstoffeinleitung wurden
- 100 g (0,25 mol) Di-(isoamylguerbet)-maleinat (hergestellt gemäß Beispiel 3),
- 24,66 g (0,13 mol) Natriumdisulfit und
- 41,31 g vollentsalztes Wasser
   vorgelegt und der Ansatz bei 104 °C unter Rückfluß und leichtem Stickstoffstrom so lange gerührt, bis eine klare Lösung entstanden war (ca. 3 Stunden). Es wurde noch 10 Minuten nachgerührt. Im Produkt war kein Sulfit mehr nachweisbar. Beim Abkühlen vergelte/verfestigte sich das Produkt.

Das so erhaltene Produkt wies folgende Kennzahlen auf: Säurezahl: 3,6 mg KOH/g; Epton: 10,8 %; Trockenrückstand: 74,5 Gew.-%; Gehalt Natriumsulfat: 1,8 Gew.-%.

### Dynamische Oberflächenspannung

Die Bestimmung der dynamischen Oberflächenspannung (OS) erfolgte mittels Blasendrucktensimetrie unter Verwendung eines Krüss Blasendrucktensiometers BP 2. Hierzu wurden wäßrige Lösungen mit einem Aktivsubstanzgehalt von 0.1 Gew.-% hergestellt, die Messung erfolgte bei 25 °C.

Die erhaltenen Werte sind in Tabelle 1 zusammengestellt. Angegeben sind neben den Daten für die Substanz des Vergleichsbeispiels B2 (Di-2-PH-SUS) und den Daten des erfindungsgemäßen Beispiels B4 (Di-isoamylguerbet-SU) zum Vergleich zusätzlich die Daten zweier weiterer Substanzen, nämlich dem Natrium-di-(2-ethylhexyl)sulfosuccinat (Di-2-EH-SUS), welches ein bewährtes Handelsprodukt ist, und dem Natrium-di-(isodecyl)sulfosuccinat (Di-isodecyl-SUS), dessen Alkoholbaustein dieselbe Anzahl von C-Atomen aufweist (nämlich zehn) wie der Alkoholbaustein der erfindungsgemäßen Verbindung gemäß Beispiel 4.

**Tabelle 1:**

| **Di-2-EH-SUS** | | **Di-2-PH-SUS (gemäß** | | **Di-isodecyl-SUS** | | **Di-isoamylguerbet-SUS (gemäß Beispiel 4)** | |
|---|---|---|---|---|---|---|---|
| **Blasen-frequenz [Hz]** | **Oberfl-spannung [mN/m]** | **Blasen-frequenz [Hz]** | **Oberfl-spannung [mN/m]** | **Blasen-frequenz [Hz]** | **Obenfl-spannung [mN/m]** | **Blasen-frequenz [Hz]** | **Oberfl-spannung [mN/m]** |
| 20.112 | 46.1 | 18.311 | 59 | 19.752 | | 19.652 | 53.6 |
| 10.203 | 40.6 | 10.118 | 50.9 | 10.081 | 65.5 | 10.261 | 46.4 |
| 3.062 | 38.8 | 3.065 | 35.9 | 2.967 | 52.9 | 3.062 | 32.4 |
| 2.026 | 38.3 | 2.013 | 32.3 | 2.024 | 49.1 | 2.016 | 30.2 |
| 0.507 | 36.1 | 0.523 | 27.0 | 0.5 | 31.7 | 0.52 | 28.0 |
| 0.256 | 35.2 | 0.254 | 26.3 | 0.249 | 29.2 | 0.25 | 27.6 |
| 0.098 | 33.8 | 0.099 | 26.1 | 0.093 | 26.9 | 0.086 | 27.1 |

Es wurde gefunden, dass das erfindungsgemäße Sulfosuccinat gemäß Beispiel 4 einerseits effektiv die statische Oberflächenspannung (OS) senkt (die OS des erfindungsgemäßen Di-isoamylguerbet-SUS bei Blasenfrequenzen von unter 0.1 Hz ist signifikant geringer als die OS des Handelsproduktes Di-2-EH-SUS) andererseits ein überraschend gutes dynamische Verhalten aufweist. So steigt die OS wäßriger Lösungen des Di-isoamylguerbet-SUS (dessen Alkoholbaustein 10 C-Atome aufweist und 3-fach verzweigt ist) erst bei Blasenfrequenzen oberhalb 2 Hz merklich und erst ab 3 Hz stark an. Beim Di-2-PH-SUS (dessen Alkoholbaustein ebenfalls 10 C-Atome aufweist, jedoch nur 1-fach verzweigt ist) ist dies schon bei 0.5 beziehungsweise 2 Hz zu beobachten. Beim Vergleich mit dem Handelsprodukt Di-2-EH-SUS (dessen Alkoholbaustein 8 C-Atome aufweist und 1-fach verzweigt ist) fällt zudem auf, dass der Anstieg der OS mit steigender Blasenfrequenz zunächst geringer ausfällt als beim erfimdgsgemäßen-Di-isoamylguerbet-SUS, welches dann allerdings bei einer Blasenfrequenz von 10 Hz einen starken Anstieg der OS aufweist. Wegen der deutlich geringeren statischen OS des erfindungsgemäßen Sulfosuccinats übersteigt die OS seiner wäßrigen Lösung erst bei einer Blasenfrequenz von 5 Hz diejenige einer wäßrigen Di-2-EH-SUS-Lösung (Di-C8V1-SUS). Da die Blasenbildungsfrequenz ein Maß für die Rate der Oberflächen-Neubildung ist, läßt sich feststellen, dass die erfindungsgemäßen Sulfosuccinate eine Verbesserung der oben beschriebenen technischen Anwendungen ermöglicht.

## Patentansprüche

1. Sulfosuccinate der allgemeinen Formel (I), 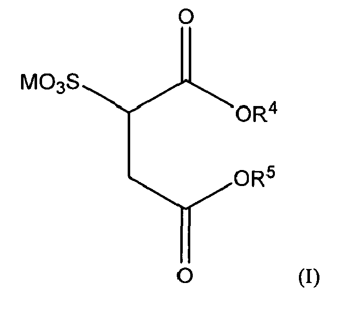 worin M Wasserstoff oder ein Kation und die Reste R⁴ und R⁵- unabhängig voneinander - Wasserstoff oder ein Kation oder eine Alkylgruppe bedeuten, wobei höchstens einer der Reste R⁴ oder R⁵ Wasserstoff oder ein Kation ist, wobei diese Sulfosuccinate mindestens einen Alkohol-Baustein enthalten, der den Monoalkoholen mit insgesamt 8 bis 36 C-Atomen zuzurechnen ist, mit der Maßgabe, dass es sich bei diesen Monoalkoholen um Guerbet-Alkohole (GA) handelt, die mindestens zwei Verzweigungen pro Molekül enthalten.

2. Sulfosuccinate nach Anspruch 1, mit der Maßgabe, dass es sich bei den Monoalkoholen um Guerbet-Alkohole (GA) handelt, die mindestens drei Verzweigungen pro Molekül enthalten.

3. Sulfosuccinate nach Anspruch 2, mit der Maßgabe, dass es sich bei den Monoalkoholen um Guerbet-Alkohole (GA) handelt, die drei Verzweigungen pro Molekül enthalten und die erhältlich sind, indem man als Ausgangsalkohole für die Guerbet-Reaktion zur Herstellung dieser Guerbet-Alkohole (GA) ausschließlich solche Monoalkohole mit 4 bis 18 C-Atomen einsetzt, die ausgewählt sind aus der Gruppe der primären und/oder sekundären Monoalkohole der Formel (MA), 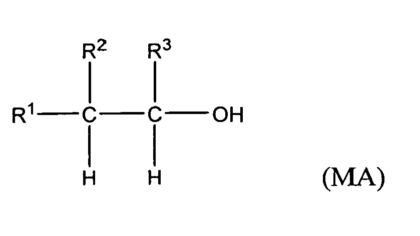 wobei gilt: (a) die Gesamtzahl der C-Atome der Verbindungen (MA) liegt im Bereich von 4 bis 18; (b) die Reste R¹, R² und R³ bedeuten Wasserstoff oder Alkylgruppen, die - unabhängig voneinander - linear oder verzweigt oder alicyclisch, gesättigt oder ungesättigt sein können; (c) die Reste R¹ und R² und/oder R¹ und R³ und/oder R² und R³ können miteinander verknüpft sein, d.h. Teil einer alicyclischen Substruktur sein; (d) die Verbindungen (MA) weisen mindestens eine Verzweigung auf.

4. Sulfosuccinate nach Anspruch 3, wobei die Monoalkohole (MA) gesättigte, mindestens einfach verzweigte Monoalkohole mit 5 bis 10 C-Atomen sind.

5. Sulfosuccinate nach Anspruch 3 oder 4, wobei es sich bei dem Monoalkohol (MA) um 3-Methyl-butan-1-ol handelt.

6. Sulfosuccinate nach einem der Ansprüche 1 bis 5, wobei M ausgewählt ist aus der Gruppe 1-wertiger Kationen.

7. Sulfosuccinate nach Anspruch 6, wobei M ausgewählt ist aus der Gruppe der Alkalimetall-Kationen.

8. Sulfosuccinate nach Anspruch 6, wobei M ein Ammoniumion ist.

9. Sulfosuccinate nach einem der Ansprüche 1 bis 8, wobei es sich um Dialkylsulfosuccinate handelt.

10. Zusammensetzungen, enthaltend ein oder mehrere Sulfosuccinate gemäß den Ansprüchen 1 bis 9 und Wasser.

11. Verwendung der Sulfosuccinate (I) gemäß den Ansprüchen 1 bis 9 als Tenside.
